# EUROPEAN PATENT APPLICATION

(11) **EP 3 815 622 A1**
(43) Date of publication of application: **05.05.2021**
(21) Application number: 19205836.0
(22) Date of filing: 29.10.2019
(51) Int. Cl.: A61B 10/02, A61B 10/04

(54) **FLEXIBLE ENDOSCOPIC TISSUE BIOPSY DEVICE**

(71) Applicant: Stichting Katholieke Universiteit, 6525 EZ Nijmegen (NL)
(72) Inventor: VERHOEVEN, Roel Lambertus Johannes, 6500 HB Nijmegen (NL); VAN DER HEIJDEN, Henricus Franciscus Maria, 6500 HB Nijmegen (NL)
(74) Representative: Nederlandsch Octrooibureau

(57) **Abstract**

A tissue biopsy device (1) with a first tubular component (2) and a second tubular component (3), the second tubular component (3) surrounding the first tubular component (2). The first tubular component (2) has a tissue piercing distal part (4) with a hollow core, and the second tubular component (3) has a distal end part (5) with a cutting surface (6). The first tubular component (2) and the second tubular component (3) are flexible in a length wise direction, allowing to use the tissue biopsy device (1) in a more versatile manner.

## Description

### Field of the invention

The present invention relates to a tissue biopsy device which can be used in procedures involving (flexible) endoscopic tools for obtaining tissue diagnosis, either through analysis of cytology or histology. More specifically, the present invention relates to a tissue biopsy device, comprising a first tubular component and a second tubular component, the second tubular component surrounding the first tubular component, the first tubular component comprising a tissue piercing distal part with hollow core, the second tubular component comprising a distal end part with a cutting surface.

### Background art

European patent publication EP-A-2 623 036 discloses an instrument for taking a tissue sample, having a spiral or helical tissue receiving element and a tubular cutting element surrounding the tissue receiving element, the tubular cutting element having a distal end provided with a sharp edge. The cannula is of a rigid, non-deformable structure. The tissue receiving element has a near constant inner opening diameter, and an outer diameter which decreases from a proximal part to a distal part of the tissue receiving element.

### Summary of the invention

The present invention seeks to enable and provide an improved device for obtaining tissue biopsy samples in flexible endoscopic procedures, as compared to currently available techniques in the field of (flexible) endoscopy.

According to the present invention, a tissue biopsy device as defined above is provided, wherein the first tubular component and the second tubular component are flexible in a length wise direction. The flexibility allows to use the tissue biopsy device together with further flexible instruments, such as endoscopes or catheters, allowing to reach a target tissue in an efficient manner. A complete through-cut tissue sample can be obtained in one go, with a size sufficient to allow both cytological and histological analysis (both molecular diagnostics and tissue diagnosis). Also the biopsy sampling is atraumatic to surrounding tissue, further enhancing efficiency of the biopsy procedure.

### Short description of drawings

The present invention will be discussed in more detail below, with reference to the attached drawings, in which
Fig. 1 shows a partial side view of a first tubular component in accordance with an embodiment of the present invention tissue biopsy device;
Fig. 2 shows a partial side view of a second tubular component in accordance with an embodiment of the present invention tissue biopsy device;
Fig. 3 shows an enlarged perspective view of a distal end part of the first tubular component; and
Fig. 4 shows an enlarged perspective view of a distal end part of the second tubular component.

### Description of embodiments

The present invention embodiments of a tissue biopsy device may be utilized to improve lung cancer diagnosis and staging, but is also relevant to other flexible endoscopic applications for tissue acquisition and characterization.

Products known to the field for obtaining tissue specimens in lung cancer through flexible endoscopy are cytology needles, brushes, forceps, and cryobiopsies. Needles herein aspirate cells through movement of the hollow needle through the tissue (possibly in combination with simultaneous application of suction through the hollow lumen), and are usable only for cytology. In terms of operating mechanisms, the brushing tool, cryoprobe and forceps all have in common that they are superficial of origin and have limited penetration depth. These tools furthermore need an already existing lumen for being able to properly operate. The (aspiration) needle does not need this. This relies on repeated movement of the needle inside the lesion (as induced by the operator) for obtaining an aspirate of cells (possibly under vacuum application to the needle lumen). Although this methodology can possibly aspirate cells both superficially as well as to a greater depth, both literature on flexible endoscopy for peripheral lesions and EBUS-TBNA/EUS-FNA of lymph nodes have shown that its diagnostic yield is far from ideal even after multiple passes. EBUS-TBNA is an acronym for Endobronchial Ultrasound - Trans Bronchial Needle Aspiration, and EUS-FNA is an acronym for endoscopic ultrasound guided fine needle aspiration. As opposed to the forceps and cryobiopsy (but similar to the brush), the hollow needle only obtains cells and does not necessarily represent a complete cross-section of the penetrated tissue. Brushes can only be used in diagnosing peripheral pulmonary lesions which are endobronchially located, and are brushed alongside the suggested lesion location for cytology. Forceps are used to grab a part of tissue and remove it by applying (traumatic) force after closure of the device. The tissue acquired by forceps is usable for histology, but often sub-optimal in assessment quality due to crushing artefacts (by its traumatic origin). Cryobiopsy involves freezing an inserted probe to -70 degrees for several seconds, after which surrounding tissue will become frozen to the probe surface and removed by forcibly pulling out the probe (traumatic) for histology specimen collection. All of these instruments are inserted through the endoscope working channel or a catheter extending outside of this endoscope working channel to facilitate localized tissue biopsy positioning. Under guidance of imaging and by means of guiding catheters extending out of flexible endoscopes, these tools are used for tissue acquisition.

Repeated sampling is necessary to maximize the chance of having adequate tissue for cyto- and histopathology. Multiple studies have shown that this hit and run technique within a lesion results in only a small amount of taken biopsies containing representative material. This is not only caused by the current tools and instruments, but also because pathologic tissue is often heterogeneous and might not necessarily all contain representative material that can be correlated to lesion origin. Repeated biopsy involves time and a lot of cost for multiple (disposable) devices. It adds complexity to the procedure. Every tool has specific properties which can be selected for a slightly different means of tissue acquisition.

Making the procedure even more difficult is that the lesion location can often be in complex relation when related to the remainder of its environment and the tools used. An example of this would be in early stage lung cancer; where only a minor lesion in the peripheral of the lung might be present. The nodule is often stiffer than surrounding tissue. Tool insertion will more easily deform surrounding tissue and also aspirate/biopsy surrounding tissue, especially if a pushing technique for tissue acquisition is used. Its position relative to the tool is very often not such that every biopsy direction will acquire suspected pathological tissue along the biopsy tool insertion direction. The nodule is often of small size, making the exact insertion position of the tool very important. Tool insertion and biopsy is often performed under no local vision (i.e. only C-arm imaging for global positioning, with limited resolution). The endoscopic tool itself has a certain rigidity which might be different than previous technology that has been used to confirm lesion position, and, higher than that of the guiding device itself. Stiffness of differently used endoscopic tools will affect tissue positioning.

Previous studies in targeting early stage lung cancer lesions for diagnosis have shown that as a consequence of biopsy tool limitations, approximately a 20% discrepancy between navigation success and diagnostic success remains. Where for example it is possible to verify through imaging that navigation was successful in approximately 90% of lesions, a diagnostic outcome corresponding to follow up outcome was only obtained in approximately 72% of cases. Obtaining quality specimens is a problem not unknown to the field of flexible endoscopy, and also remains of interest in for example endoscopic mediastinal lymph node staging.

Obtaining accurate and representative tissue biopsy samples may be greatly improved by using any one of the present invention embodiments, according to which a tissue biopsy device is provided, comprising a first tubular component 2 and a second tubular component 3, the second tubular component 3 surrounding the first tubular component 2. The first tubular component 2 comprises a tissue piercing distal part 4 with a hollow core, and the second tubular component 3 comprises a distal end part 5 with a cutting surface 6, e.g. in the form of a sharp (circular) edge or cutting element extending from the edge. The first tubular component 2 and the second tubular component 3 are flexible in a length wise direction. The flexibility of the components 2, 3 of the tissue biopsy device 1, i.e. the capability to bend in directions perpendicular to a longitudinal axis of the first and second tubular component 2, 3, allows to use the tissue biopsy device 1 in combination with a flexible endoscopic device or the like, allowing to more easily and precisely obtain tissue samples. It is noted that the distal part 4 is at least partially provided with the hollow core to enable capturing a biopsy sample. The hollow core can in further embodiments extend along the entire distal part 4 or even into and along the first tubular component 2. In further embodiments, the first tubular component 2 and/or the second tubular component 3 are torsionally stiff], allowing to reliably rotate the first and second tubular components 2, 3 in unison, or independently from each other, during actual use of the tissue biopsy device 1.

Once the distal part of the tissue biopsy device 1 is at a target location, the tissue piercing distal end 4 (with its hollow core) of the first tubular component 2 can be rotated to pierce an amount of tissue, after which the second tubular component 3 can be rotated over the first tubular component 2 to cut through the tissue with the cutting surface 6. The tissue biopsy device 1 can then be retracted with the biopsy sample contained in the (hollow core) of the distal part thereof.

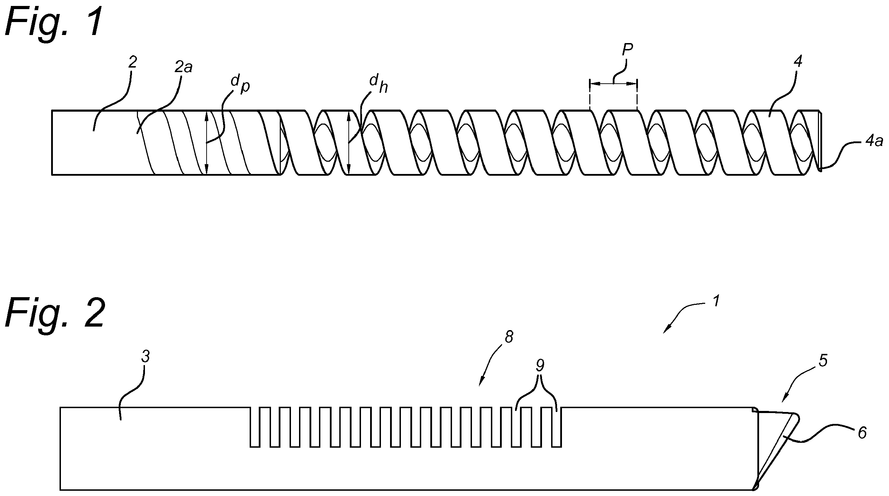

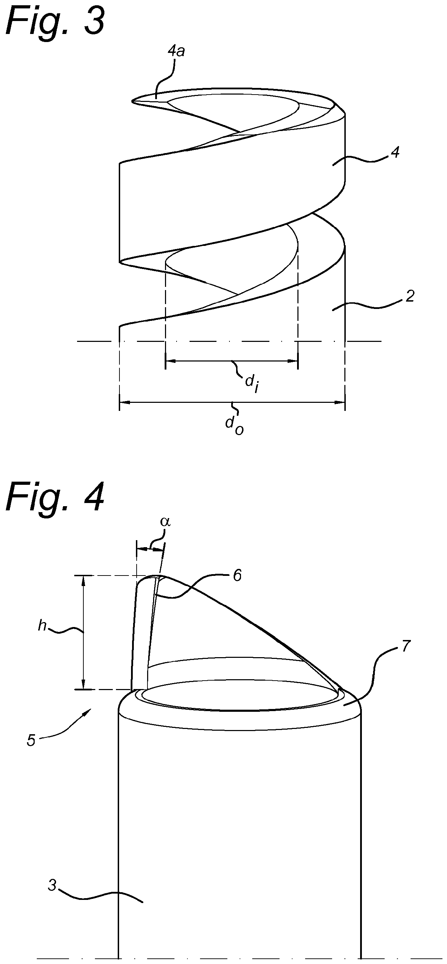

A partial side view of the first tubular component 2 is shown in Fig. 1, and a partial side view of the second tubular component 3 is shown in Fig. 2. Furthermore, Fig. 3 shows an enlarged perspective view of a distal end part of the first tubular component 2, and Fig. 4 shows an enlarged perspective view of a distal end part of the second tubular component 3.

In Fig. 1, the first tubular component 2 is shown, with the (operative) distal part thereof, comprising a tissue piercing distal end 4 having a sharp end face 4a at a distal end of the tissue piercing distal part 4. The sharp end face 4a is non sharp along the insertion direction, but arranged to penetrate tissue by rotation of the sharp end face 4a into the tissue. In the exemplary embodiment shown, the tissue piercing distal end 4 has a helical shape with a hollow core, with a pitch distance P. The tissue piercing distal part 4 has a pitch distance P of between 0.1 and 5 mm which allows proper piercing action in combination with a proper holding force of the tissue biopsy sample for consecutive shear force based cutting of tissue using the second tubular component 3. A single (uniform) or varying pitch distance may be provided in further embodiments of the present invention. Also shown in Fig. 1 is the outer diameter dₕ of the tissue piercing distal end 4, and the outer diameter dₚ of a proximal part 2a of the first tubular component 2. As exemplary embodiments, the outer diameter dₕ of the tissue piercing distal end 4 may be between 0.4 and 4 mm, and the outer diameter dₚ of the proximal part 2a may be between 0.1 and 5 mm.

In a group of embodiments of the tissue biopsy device according to the present invention, the tissue piercing distal part 4 has a helical shape, as shown in the exemplary embodiment shown in Fig. 1. Alternatively, the shape of the tissue piercing distal part 4 can be in the form of a spiral, a coil, etc. which are also providing a unidirectional piercing action to the second tubular component 3 when being rotated during use.

As shown in the detail perspective view of Fig. 3, the end part of the tissue piercing distal part 4 is provided with a sharp end face 4a, e.g. a sharpened end surface or sharp point, which in use is able to pierce through tissue in a atraumatic manner. In the perspective view of Fig. 3, it is even more clearly shown that the tissue piercing part 4 is provided with a hollow core, showing the internal diameter dᵢ of the tissue piercing distal part 4 and the outer diameter dₒ. The internal diameter dᵢ can range between 0.1 and 4 mm, the outer diameter dₒ of course larger than the inner diameter dᵢ.The hollow core ensures that sufficient tissue is available with the biopsy sample, which is not touched by the tissue biopsy device at all, thus allowing both cyto- and histopathology on the obtained sample.

It is noted that in the exemplary embodiment shown in Fig. 1 and 3, the sharp end face 4a comprises a shaped end surface which is sharp as seen in one rotational direction of the tissue piercing distal part 4, but flat as seen in an opposite rotational direction of the tissue piercing distal part 4. This provides for an atraumatic end when the distal end part 4 is not rotated or rotated in the correct (atraumatic) direction, as the then flat shape of the sharp end face 4a will ensure not to penetrate tissue with the tissue biopsy device 1. As the distal end part 4 is rigidly connected to the first tubular component 2 there will be no tissue penetration when the first tubular component 2 is not turned, e.g. when travelling to a target location, or when the first tubular component 2 is turned in a non-penetrating direction, and there will be tissue penetration when turned in the one rotational direction of the tissue piercing distal part 4.

Fig. 2 shows a side view of an exemplary embodiment of the second tubular component 3, which in operation, surrounds the first tubular component 2. At a distal end part 5 of the second tubular component, a cutting surface 6 is provided. Furthermore, in order to provide the second tubular component 3 with additional flexibility, it comprises a patterned tube wall 8. In the embodiment shown, the patterned tube wall 8 comprises a plurality of slits 9, providing an enhanced flexibility predominantly in one direction.

In an exemplary embodiment, the slits 9 are provided on an arc section of the patterned tube wall 8, e.g. over 180 degrees, or even more. This allows bending in the direction of the second tubular component 3 where the slits 9 are provided. The slits 9 can alternatively also be provided in a staged manner, i.e. with a different arc direction for adjacent slits, providing a bending capability in more directions. As an even further embodiment, the slits 9 can be provided as helical or screw thread type slit(s) spanning multiple times the circumference of the second tubular component 3.

As an alternative embodiment, the patterned tube wall 8 comprises a braided material tube wall. Braided material can be provided in a tubular form, and having torsional stiffness, by proper selection of the material used and the braiding pattern. In general, such a patterned tube wall 8 of braided material provides a flexibility to bend in all directions perpendicular to a longitudinal axis of the second tubular component 3.

In an even further advantageous embodiment, the first tubular component 2 comprises a hollow core, and the tissue biopsy device further comprises a flexible component positioned within the hollow core. The flexible component is e.g. a pull wire, a guide wire or a steerable flexible endoscopic device.

To enhance controllability of the flexing of the tissue biopsy device 1, a pull wire, guide wire or steerable flexible endoscope device may be provided, which is attached near or at the distal end part 5 of the second tubular component 3. The point where the pull wire is attached then determines the direction of bending of the second tubular component 3 (and the first tubular component 2 inside thereof).

If the tissue biopsy device further comprises a guide wire as a flexible component in a further embodiment, the guide wire being positioned within the hollow core of the first tubular component 2, this enables, during actual use, a more precise positioning of the distal end of the tissue biopsy device 1, or enables a more simple routing of the tissue biopsy device 1 towards the target tissue.

The detail perspective view of Fig. 4 shows an exemplary embodiment of the cutting surface 6 provided at a rim 7 of the second tubular component 3. In the embodiment shown, the cutting surface 6 is inclined from a rim 7 of the distal end part 5 towards a center line of the second tubular component 3. In the embodiment shown, the cutting surface 6 inclines inward with an angle α from the outer wall surface of the second tubular component 3. As long as the tissue piercing end part 4 of the first tubular component 2 is kept below the rim 7 of the second tubular component 3, the cutting surface 6 will be directed inwardly, avoiding interference with a secondary internal wall of an endoscope, catheter, extended working channel or other similar devices as used in operation. The cutting surface 6 is pushed outwards by the first tubular component 2, more specifically by the tissue piercing distal part 4, allowing a tight fit for a neat cutting action (by shear force) in combination for resecting the desired biopsy sample. Note that the cutting surface 6 can alternatively or additionally be bendable with respect to the wall of second tubular component 3, e.g. made of a memory material such as nitinol, to ensure proper functioning in combination with the tissue piercing distal part 4.

The embodiment shown in Fig. 4 is also a nice example of a further group of embodiments, wherein the cutting surface 6 is operative only in one rotational direction of the second tubular component 3. This allows the distal end of the second tubular component 3 (and the entire tissue biopsy device 1) to be atraumatic when rotated in the other, non-cutting direction. A height h of the cutting surface 6 is designed to be higher than the pitch distance P of the tissue piercing distal part 4, to ensure proper functioning of both the tissue piercing distal part 4 and the cutting surface 6.

Of course, alternative implementations can be envisaged for the cutting surface 6, e.g. by providing more than one cutting surface 6. Alternatively, the cutting surface 6 is a sharpened circumferential surface of the rim 7. The sharpened circumferential surface may also comprise a sawtooth type of profile.

In a further group of embodiments, the first tubular component 2 comprises a proximal part 2a having an outer diameter dₚ which is less than an outer diameter dₕ of the tissue piercing distal part 4 (see also the embodiment shown in Fig. 1). By having a (slightly) smaller diameter of the proximal part 2a, rotation and other mutual movement between the first and second tubular components 2, 3 is improved. The outer diameter dₕ of the tissue piercing distal part 4 is e.g. in the order of 2 mm, while the outer diameter dₚ of the proximal part 2a is in the order of 1.9 mm or less.

The tissue piercing distal part 4 and proximal part 2a of the first tubular component may comprise different materials, which may be attached to each other using known attachment techniques (gluing, welding, melting, etc.). The material properties can then be exploited to enhance the respective function of both the tissue piercing distal part 4 (penetrating tissue) and the proximal part 2 (relaying longitudinal and rotational forces).

For further improvements for users during actual application of the tissue biopsy device 1, in a further group of embodiments, the tissue piercing distal part 4 comprises fluoroscopic opaque material. This allows to provide clear and live images during a biopsy, using readily available imaging techniques such as X-ray fluoroscopy. The entire tissue piercing distal part 4 may be made of fluoroscopic opaque material, or separate markers can be attached thereto.

To assist the user of the present invention embodiments of the tissue biopsy device 1, one or more positioning markers are provided in a further embodiment, on respective proximal parts of the first tubular component 2 and/or second tubular component 3. This provides a correct and actual indication of mutual positioning of the distal parts of the first and second tubular components 2, 3, and/or positioning with respect to a further device, e.g. an endoscope tube.

The tissue acquired with any one of the present invention embodiments described herein is a solid core of tissue, obtained in an atraumatic manner, thereby removing the chance of any crushing artefacts disabling pathologic analysis. As a (cylindrical) solid core is an adequate representation of tissue over multiple depths of the nodule in a single shot and with sufficient thickness when related to total lesion size, diagnostics become more systematic and representative. Rather than a hit and run approach within a lesion, a single representative cross-section of tissue is obtained that allows for greater systematic analysis by the pathologist. This biopsy technique may replace the need for (repeated) needle aspiration, brushing, forceps biosy and cryobiopsy, and can provide an accurate diagnostic and systematic staging tool. Especially in early stage lung cancer, it can be used in combination with presently available navigation guidance or imaging technology. Its specific design in this application further enables an immediate α-traumatic resection for a consecutive trans-parenchymal biopsy whereas tools previously known to the field require secondary tools to do so. Again, using the present invention embodiments, it is foreseen that it is possible to reduce needed biopsy tools and time, increasing diagnostic success at the same time.

The present invention embodiments, as described above with reference to the exemplary embodiments shown in the figures, and as described in the attached claims, provide a number of distinct advantages over other (prior art) techniques:
- A complete through-cut specimen of tissue is provided in one go, unique in size when related to tissue biopsy device 1 diameter.
- Both molecular diagnostics and tissue diagnosis are enabled because of sufficient sample size.
- The biopsy sampling is α-traumatic to surrounding tissue as it works according to a self-containing mechanism. The helical structure of the tissue piercing distal part 4 with hollow core pierces the tissue by rotation. By its mechanism of action, the tissue piercing distal part 4 anchors itself into tissue. After having anchored into the lesion, a secondary cutting action is accomplished using the second tubular component 3, which is moved over the helical structure of the tissue piercing distal part 4 to subsequently extract the tissue piercing distal part 4 with said tissue sample. All needed force for extraction resides at the boundary interaction of both devices rather than surrounding lung tissue or inside the hollow core containing the tissue specimen.
- The hollow core can be used to accommodate for secondary (guiding) devices such as a guiding wire as described above. This further facilitates a workflow where more exact positioning of tools can be made to obtain a tissue biopsy sample.
- The tissue biopsy device 1 is atraumatic upon insertion through the endoscope and when pushed against tissue.
- The distal parts (sharp end face 4a of tissue piercing distal part 4 and rim 7/cutting surface 6 of the distal part 5) do not induce trauma when rotated counter-directional to the helical structure of the tissue piercing distal part 4.
- The distal parts only induce trauma (tissue piercing) when rotated along the direction of the helical structure of the tissue piercing distal part 4.
- As only a single tissue acquisition is needed for every nodule or lymph node, more time can be spent for precise positioning.
- The helical structure of the tissue piercing distal part 4 allows for high flexibility of the entire tissue biopsy device 1 relative to its diameter and biopsy size. The deformation of tissue in-vivo caused by the tissue biopsy device 1 will not significantly alter positioning of the distal parts thereof

### (i.e. this is a big problem in cryobiopsy and forceps use in peripheral nodules)

- The tissue biopsy device 1 pulls tissue inside itself through a unique motion (rotation). This enables a further unique interaction of the tissue biopsy device 1 with relation to the lesion. A rotation for a first anchorage inside the tissue can be accompanied by a secondary pushing/pulling. By these means, even though initial angulation is not ideal, the lesion can be stretched out in front of the tool and consecutively pierced by a rotating motion.
- The hollow inner core of the tissue piercing distal part 4 can possibly accompany a sharp guidewire facilitating trans-parenchymal access of the devices, whereas previous tools needed a sequential approach of the pulmonologist through multiple devices to do so.

By its unique design, the present invention embodiments of the tissue biopsy device 1 may serve as a single shot diagnostic mechanism/device that is able to be used in all cases in a simplistic and verified fashion. Because it is a do-it-all device, a further advantage is that the procedural workflow complexity is reduced. By doing so, physicians will not need to decide on the to-be-used product in different cases as one replaces all, decreasing learning curve and increasing overall procedure performance. By being successful, the sensitivity of procedures might increase by as much as 16 to 20% ((EBUS/EUS and peripheral nodules, respectively), based on previous literature and studies. Concluding, by this invention, diagnostic yield should become equal to navigation success.

The present invention has been described above with reference to a number of exemplary embodiments as shown in the drawings. Modifications and alternative implementations of some parts or elements are possible, and are included in the scope of protection as defined in the appended claims.

## Claims

1. A tissue biopsy device, comprising
a first tubular component (2) and a second tubular component (3), the second tubular component (3) surrounding the first tubular component (2),
the first tubular component (2) comprising a tissue piercing distal part (4) with a hollow core,
the second tubular component (3) comprising a distal end part (5) with a cutting surface (6),
wherein the first tubular component (2) and the second tubular component (3) are flexible in a length wise direction.

2. The tissue biopsy device according to claim 1, wherein the first tubular component (2) and/or the second tubular component (3) are torsionally stiff.

3. The tissue biopsy device according to claim 1 or 2, wherein the cutting surface (6) is inclined from a rim (7) of the distal end part (5) towards a center line of the second tubular component (3).

4. The tissue biopsy device according to any one of claims 1-3, wherein the cutting surface (6) is operative only in one rotational direction of the second tubular component (3).

5. The tissue biopsy device according to any one of claims 1-4, wherein the tissue piercing distal part (4) has a helical shape.

6. The tissue biopsy device according to any one of claims 1-5, wherein the second tubular component (3) comprises a patterned tube wall (8).

7. The tissue biopsy device according to claim 6, wherein the patterned tube wall (8) comprises slits (9).

8. The tissue biopsy device according to claim 7, wherein the slits (9) are provided on an arc section of the patterned tube wall (8).

9. The tissue biopsy device according to any one of claims 1-5, wherein the patterned tube wall (8) comprises a braided material tube wall.

10. The tissue biopsy device according to any one of claims 6-9, wherein the first tubular component (2) comprises a hollow core, the tissue biopsy device further comprising a flexible component positioned within the hollow core.

11. The tissue biopsy device according to any one of claims 1-10, wherein the first tubular component (2) comprises a proximal part (2a) having an outer diameter (dₚ) which is less than an outer diameter (dₕ) of the tissue piercing distal part (4).

12. The tissue biopsy device according to claim 11, wherein the tissue piercing distal part (4) and proximal part (2a) comprise different materials.

13. The tissue biopsy device according to any one of claims 1-12, wherein the tissue piercing distal part (4) comprises a sharp end face (4a).

14. The tissue biopsy device according to claim 13, wherein the sharp end face (4a) comprises a shaped end surface which is sharp in one rotational direction of the tissue piercing distal part (4) and atraumatic in an opposite rotational direction of the tissue piercing distal part (4).

15. The tissue biopsy device according to any one of claims 1-14, wherein the tissue piercing distal part (4) has a pitch distance (P) of 0.1-5 mm.
